# FASCICULE DE BREVET EUROPEEN

(11) **EP 0 745 122 B1**
(45) Date de publication et mention de la délivrance du brevet: **19.11.2008**
(21) Numéro de dépôt: 95909825.2
(22) Date de dépôt: 14.02.1995
(51) Int. Cl.: C12N 15/12, C07K 14/72, C12N 15/11, C12Q 1/68, C12N 5/10, G01N 33/74, C07K 14/575, A61K 38/22

(54) **RECEPTEUR GALANINE, ACIDES NUCLEIQUES, CELLULES TRANSFORMEES ET UTILISATIONS**
GALANINREZEPTOR, NUKLEINSÄUREN,TRANSFORMIERTE ZELLEN UND VERWENDUNGEN
GALANIN RECEPTOR, NUCLEIC ACIDS, TRANSFORMED CELLS AND USES THEREOF

(30) Priorité: 17.02.1994 FR 9401808
(43) Date de publication de la demande: 04.12.1996
(73) Titulaire: Aventis Pharma S.A., 92160 Antony (FR); INSTITUT NATIONAL DE LA SANTE ET DE LA RECHERCHE MEDICALE (INSERM), 75654 Paris Cédex 13 (FR)
(72) Inventeur: AMIRANOFF, Brigitte, F-91390 Morsang-sur-Orge (FR); HABERT-ORTOLI, Estelle, F-75011 Paris (FR); LOQUET, Isabelle, F-92160 Antony (FR)
(86) Numéro de dépôt international: PCT/FR1995/000172
(87) Numéro de publication internationale: WO 1995/022608

(56) Documents cités:
- EP-A- 0 514 361
- WO-A-92/15015
- WO-A-94/00590
- BRAIN RESEARCH, vol.625, no.1, 15 Octobre 1993, AMSTERDAM, PAYS BAS pages 173 - 176 A. HULTING ET AL. 'Galanin receptors from human pituitary tumors assayed with human galanin as ligand.'
- PROCEEDINGS OF THE NATIONAL ACADEMY OF SCIENCES OF THE USA, vol.90, no.23, 1 Décembre 1993, WASHINGTON DC, ¹TATS UNIS pages 11287 - 11291 T. BARTFAI ET AL. 'Galanin-receptor ligand M40 peptide distinguishes between putative galanin-receptor subtypes.'
- BAILLIÈRE'S CLINICAL ENDOCRINOLOGY AND METABOLISM, vol.8, no.1, Janvier 1994, LONDRES, GRANDE BRETAGNE pages 77 - 110 M. LABURTHE ET AL. 'Receptors for gut regulatory peptides.'
- THE JOURNAL OF BIOLOGICAL CHEMISTRY, vol.264, no.34, 5 Décembre 1989, BALTIMORE MD, ¹TATS UNIS pages 20714 - 20717 B. AMIRANOFF ET AL. 'Galanin receptor in the rat pancreatic beta cell line Rin m 5F.'
- PROCEEDINGS OF THE NATIONAL ACADEMY OF SCIENCES OF THE USA, vol.90, no.9, 1 Mai 1993, WASHINGTON DC, ¹TATS UNIS pages 3845 - 3849 Y. CHEN ET AL. 'Purification of a galanin receptor from pig brain.' cité dans la demande
- PROCEEDINGS OF THE NATIONAL ACADEMY OF SCIENCES OF THE USA, vol.91, no.21, 11 Octobre 1994, WASHINGTON DC, ¹TATS-UNIS pages 9780 - 9783 E. HABERT-ORTOLI ET AL. 'Molecular cloning of a functional human galanin receptor.'

### SEQUENCE LISTING

<110> Aventis Pharma S.A.
<120> Récepteur galanine, acides nucléiques, cellules transformées et utilisations.
<130> ST94008
<160> 2
<170> PatentIn version 3.3
<210> 1
   <211> 3083
   <212> DNA
   <213> Homo sapiens
<220>
   <221> CDS
   <222> (787)..(1836)
   <223> /produit= "recepteur galanine humain"
<400> 1
   attcggcggc gacccatccc gctagaatcc gtccagtctc tgctcgcgca ccgtgacttc     60
   taaggggcgc ggatttcagc cgagctgttt tcgcctctca gttgcagcag agaagcccct    120
   ggcacccgac tctatccacc accaggaagc ctcccaaaag agctctcgcc ctgtggacga    180
   ctcggaatcc ctggaaaagc cgggagggag tcggaggcgc cagcccactg gggaggtggc    240
   gctgggcgcg cgggatgcgc ggggagcctt ctctgcagga gccgcacagt gcactgctgc    300
   gcgctgggca gtgcggggaa gcgccgcggg aaggagcggc tccgagcaac aggtgcagca    360
   cgcagccgct ccgggagcca gggaaaaccg ccggcgaaga tctggagcgg taaggcggag    420
   agaagggtct ttccacctgc gcggctgcag ccggcggatc cctcttccca ggctccgtgg    480
   tcgcgcagcg ggcggaggcg cccgggcagg ggaccccagt gctctcgaga tcaccgtccc    540
   ttcccgagaa ggtccagctc cgggctcccg aacccaccct ctctcagaag gtcgcggcgc    600
   aaagacggtg ccaccaggca cggccaccgg atccccgctc ccgctggctc gcgcctcggg    660
   ggaagctcag actcctaaac tcgcactctc cgtgctttgc gccgggaccc ctggccaccc    720
   ccggcgcctg ctatcccgcc ctccctcccg cgcgctccgc cgctcgccgg gacagccccg    780
   cgggcc atg gag ctg gcg gtc ggg aac ctc agc gag ggc aac gcg agc        828
   tgt ccg gag ccc ccc gcc ccg gag ccc ggg ccg ctg ttc ggc atc ggc       876
   gtg gag aac ttc gtc acg ctg gtg gtg ttc ggc ctg atc ttc gcg ctg       924
   ggc gtg ctg ggc aac agc cta gtg atc acc gtg ctg gcg cgc agc aag       972
   ccg ggc aag ccg cgg agc acc acc aac ctg ttc atc ctc aac ctg agc      1020
   atc gcc gac ctg gcc tac ctg ctc ttc tgc atc ccc ttc cag gcc acc      1068
   gtg tac gcg ctg ccc acc tgg gtg ctg ggc gcc ttc atc tgc aag ttc      1116
   atc cac tac ttc ttc acc gtg tcc atg ctg gtg agc atc ttc acc ctg      1164
   gcc gcg atg tcc gtg gac cgc tac gtg gcc atc gtg cac tcg cgg cgc      1212
   tcc tcc tcc ctc agg gtg tcc cgc aac gcg ctg ctg ggc gtg ggc tgc      1260
   atc tgg gcg ctg tcc att gcc atg gcc tcg ccc gtg gcc tac cac cag      1308
   ggc ctc ttc cac ccg cgc gcc agc aac cag acc ttc tgc tgg gag cag      1356
   tgg ccc gac cct cgc cac aag aag gcc tac gtg gtg tgc acc ttc gtc      1404
   ttc ggc tac ctg ctg ccg ctc ctg ctc atc tgc ttc tgc tat gcc aag      1452
   gtc ctt aat cac ttg cat aaa aag ttg aag aac atg tca aag aag tct      1500
   gaa gca tcc aag aaa aag act gca cag aca gtt ctg gtg gtg gtt gtg      1548
   gtg ttt gga atc tcc tgg ctg ccg cac cac atc atc cat ctc tgg gct      1596
   gag ttt gga gtt ttc ccg ctg acg ccg gct tcc ttc ctc ttc aga atc      1644
   acc gcc cac tgc ctg gcg tac agc aat tcc tcc gtg aat cct atc att      1692
   tat gca ttt ctc tct gaa aat ttc agg aag gcc tat aaa caa gtg ttc      1740
   aag tgt cac att cgc aaa gat tca cac ctg agt gat act aaa gaa aat      1788
   aaa agt cga ata gac acc cca cca tca acc aat tgt act cat gtg tga      1836
   taaaagatag agtatcctta tggttgagtt tccatataag tggaccagac acagaaacaa   1896
   acagaatgag ctagtaagcg atgctgcaac ttgttatctt aacaagaatt caagtcgttt   1956
   taattaaatc ccacgtgtgt taaaaagtac tttgatccat ttaggaaatt cctaggtcta   2016
   gtgagaatta tttttcaatt ttattttagt tctaaattat gtttcagaaa caaaagacaa   2076
   tgctgtacag ttttattcct cttcagacat gaaagggaac atatatattc catatatatg   2136
   ttcaactctt catagattgt gagctggccc atcaatatgg tcaggaatat ttgcagtcta   2196
   cattttaaag ccaatttatt tagaaaaaaa atttgagctt taattcttta attttaagag   2256
   aagtaatatt gtgagctatg tattttaaaa tatgatcatg gacacacaat gatgaatttt   2316
   ttggccattt acatagacat atctattaag tggaaagaag gctttctgaa gtctgtttgc   2376
   acaggtggca tttgcttcca attgtagcta gcgcacagag ctttggaagc ctgtcattat   2436
   gagatacagt cggtttacct caggagtcaa ttcagtgttg tactggtgac ctgggatgca   2496
   gtagtaggca ctgttgattc aaatttatcc tgtgaaactg gctttataga gttaacaaaa   2556
   cagagtcaga gaccactgtc ttaacagtgg aagatgcaaa taagtttttg agaataaaac   2616
   tggattttga aattttacat tagtacttga caaaagtttt cattttgcct tgaatggaac   2676
   ctactaaaaa gagagatgaa aaaaaatcag cgaggttgat gtagataata atttctatgg   2736
   gaccaaagac tagacagaat tcagtaagtc acatgaagta atggtcatgc ctgtacataa   2796
   agcatatttc atgtttgatt tagatgacat tcaaaaaaaa tcatgggact gaatatacct   2856
   ggggtatcct atcttgtaca aatgcatgct ttttcattaa atttgtaatg atgtttaatg   2916
   aacatttcca ccaaacatta tttcctctaa aaatgttaat ttggggttaa aaccatcacc   2976
   atttgaattt caaatgtagt tttcatgaca attttatatt ggatgtgtgt ttacaatgag   3036
   aaaatggcat gaaaatatta aattgtcttg tatcggaaaa ggaattc                 3083
<210> 2
   <211> 349
   <212> PRT
   <213> Homo sapiens
<400> 2

## Description

La présente invention concerne de nouveaux polypeptides et le matériel génétique permettant leur expression. Plus particulièrement, elle concerne de nouveaux polypeptides ayant une activité de récepteur galanine.

La galanine est un neuropeptide ubiquitaire de 29 acides aminés chez les mammifères (30 chez l'homme) qui contrôle des fonctions biologiques variées : (i) secrétions endocrines (insuline, somatostatine, glucagon, hormone de croissance ...), (ii) tonicité musculaire dans le tractus digestif, (iii) contrôle du comportement (prise alimentaire, nociception, apprentissage, mémoire, douleur...) par effet neuro-modulateur au niveau du système nerveux central. Cette liste non exhaustive des effets de la galanine, mis en évidence le plus souvent chez l'animal, explique l'intérêt croissant des pharmacologues pour ce neuropeptide. Des molécules sélectives (agoniste ou antagoniste de la galanine) constitueraient des agents pharmacologiques potentiels en endocrinologie, en neurologie et en psychiatrie (Bartfai et al., (1992) TIPS 13, 312-317).

L'étude du mécanisne d'action de la galanine montre qu'elle agit par l'intermédiaire de récepteurs membranaires spécifiques. La caractérisation biochimique et moléculaire du récepteur (Chen et al., (1993) PNAS 90, 3845-3849, Fisone et al., (1989) Eur. J. Biochem. 180, 269-276) indique qu'il appartient à la famille des récepteurs couplés aux protéines G. Selon les tissus cibles, le peptide inhibe l'adénylate cyclase, diminue le calcium intracellulaire, bloque les canaux calciques voltage-dépendant ou active les canaux potassiques ATP sensibles. Des études de relations structure-activité, à l'aide de fragments C et N-terminaux de la galanine et de peptides chimériques ont montré que, (1) quelque soit le tissu, aucune séquence partielle du peptide n'est suffisante pour obtenir une activité totale, (2) selon les tissus, les 2 premiers acides aminés N-terminaux et le domaine C-terminal 16-29 de la galanine ne sont pas toujours essentiels à son activité. Ces observations suggèrent l'existence de sous-types de récepteurs galaninergiques.

La présente invention décrit pour la première fois le clonage d'un gène codant pour un récepteur galaninergique humain. La présente invention décrit également pour la première fois la séquence de récepteurs galaninergiques et leur expression dans des cellules recombinantes. La présente invention permet ainsi une meilleure compréhension de la structure des récepteurs galaninergiques, et d'étudier de manière plus fine leur mécanisme d'action. La présente invention permet également d'obtenir des récepteurs galaninergiques de très grande pureté et en quantité élevée, permettant la réalisation d'études fonctionnelles et pharmacologiques la fabrication d'anticorps, etc. L'invention permet aussi de préparer des fragments de récepteurs galaninergiques de taille définie, ainsi que toutes sortes de dérivés des récepteurs galaninergiques. L'invention fournit également des cellules recombinantes exprimant des récepteurs galaninergiques ou des fragments de récepteurs galaninergiques, utilisables pour le criblages de ligands de ces récepteurs (agonistes, antagonistes, modulateurs, etc). Les séquences d'ADN de l'invention permettent également la réalisation de sondes, capables de détecter dans des échantillons biologiques toute irrégularité dans l'expression d'un récepteur galaninergique (non-expression, mutation, polymorphisme, etc). Ces sondes sont également utilisables pour le clonage par hybridation de tout autre ADNc codant pour un récepteur galaninergique, à partir de tissus d'origines diverses, comme indiqué plus loin.

Un premier objet de l'invention réside donc dans une séquence nucléotidique codant pour un polypeptide ayant une activité de récepteur galaninergique. Au sens de l'invention, récepteur galaninergique comprend notamment tous les sous-types potentiels.
La séquence nucléotidique selon l'invention est choisie parmi :
(a) tout ou partie de la séquence nucléotidique SEQ ID n°1, et
(b) les séquences dérivées des séquences (a) en raison de la dégénérescence du code génétique.

Un mode de réalisation tout particulier de l'invention est représenté par une séquence nucléotidique comprenant tout ou partie de la séquence nucléotidique SEQ ID n°1.

Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues par exemple par criblage de banques d'ADN (banque d'ADNc, banque d'ADN génomique) au moyen de sondes élaborées sur la base de la séquence SEQ ID n°1. De telles banques peuvent être préparées à partir de cellules de différentes origines par des techniques classiques de biologie moléculaire connues de l'homme du métier. Les séquences nucléotidiques de l'invention peuvent également être préparées par synthèse chimique, notamment selon la méthode des phosphoramidites, ou encore par des méthodes mixtes incluant la modification chimique ou enzymatiqe de séquences obtenues par criblage de banques.

Les séquences nucléotidiques de l'invention peuvent être utilisées pour la production des polypeptides galaninergiques. Le terme polypeptide galaninergique désigne tout polypeptide ayant une activité de récepteur galaninergique, et tout fragment ou dérivé d'un tel polypeptide. Pour la production de polypeptides galaninergique, la partie codant pour ledit polypeptide est généralement placée sous le contrôle de signaux permettant son expression dans un hôte cellulaire. Le choix de ces signaux (promoteurs, terminateurs, etc) peut varier en fonction de l'hôte cellulaire utilisé. A cet effet, les séquences nucléotidiques de l'invention peuvent faire partie d'un vecteur, qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur. Les hôtes cellulaires utilisables pour la production des polypeptides galaninergiques de l'invention par voie recombinante sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces*, *Kluyveromyces*, *Pichia, Schwanniomyces,* ou *Hansenula.* S'agissant de cellules animales, on peut citer les cellules COS, CHO, C127, NIH-3T3, etc. Parmi les champignons, on peut citer plus particulièrement *Aspergillus* ssp. ou *Trichoderma* ssp. Comme hôtes procaryotes, on préfère utiliser les bactéries suivantes *E. coli*, *Bacillus*, ou *Streptomyces*.

Les séquences nucléotidiques de la présente invention sont également utilisables dans le domaine pharmaceutique, soit pour la réalisation de séquences antisens utilisables dans le cadre d'une thérapie génique, soit encore pour la réalisation de sondes permettant la détection, par des expériences d'hybridation, de l'expression de récepteurs galaninergiques dans des échantillons biologiques et la mise en évidence d'anomalies génétiques (polymorphisme, mutations) ou d'expressions aberrantes.

L'inhibition de l'expression de certains gènes par des séquences antisens s'est avérée être une stratégie prometteuse dans le contrôle de l'activité d'un gène. Les séquences antisens sont des séquences dont le produit de transcription est complémentaire du brin codant d'un gène donné, et est de ce fait capable d'hybrider spécifiquement avec l'ARNm transcrit ou avec le gène, inhibant sa transcription ou sa traduction en protéine. L'invention a ainsi pour objet les séquences antisens capables d'inhiber au moins partiellement la production de polypeptides galaninergiques tels que définis précédemment. De telles séquences peuvent être constituées par tout ou partie des séquences nucléotidiques définies ci-avant. Il s'agit généralement de séquences ou de fragments de séquences complémentaires de séquences codant pour des peptides de l'invention. De telles séquences peuvent être obtenues à partir de la séquence SEQ ID n°1, par fragmentation, etc, ou par synthèse chimique.

Comme indiqué ci-dessus, l'invention permet également la réalisation de sondes nucléotidiques, synthétiques ou non, capables de s'hydrider avec les séquences nucléotidiques définies ci-avant qui codent pour des polypeptides galaninergiques de l'invention, ou avec les ARNm correspondants. De telles sondes peuvent être utilisées *in vitro* comme outil de diagnostic, pour la détection de l'expression d'un récepteur galaninergique, ou encore pour la mise en évidence d'anomalies génétiques (mauvais épissage, polymorphisme, mutations ponctuelles, etc). Compte tenu des activités multiples des ligands endogènes des récepteurs galaninergiques, les sondes de l'invention peuvent ainsi permettre d'identifier des affections neurologique, cardiovasculaire, endocrinologique ou psychiatrique. Ces sondes peuvent également être utilisées pour la mise en évidence et l'isolement de séquences d'acides nucléiques homologues codant pour des polypeptides galaninergiques tels que définis précédemment, à partir d'autres sources cellulaires et préférentiellement de cellules d'origines humaines. Bien que la présente demande soit plus particulièrement illustrée par un clone désigné GalR1, les études biochimiques et immunologiques décrites dans la littérature indiquent l'existence de sous-types de récepteurs galaninergiques. Les sondes de l'invention permettent d'isoler par des techniques connues de l'homme du métier (Cf exemple 4) ces différents sous-types. Les sondes de l'invention comportent généralement au moins 10. bases, et elles peuvent comporter jusqu'à l'intégralité de la séquence SEQ ID n°1 ou de son brin complémentaire. Préférentiellement, ces sondes sont, préalablement à leur utilisation, marquées. Pour cela, différentes techniques connues de l'homme du métier peuvent être employées (marquage radioactif, enzymatique, etc). Les conditions d'hybridation dans lesquelles ces sondes peuvent être utilisées sont indiquées dans les techniques générales de clonage ci-après ainsi que dans les exemples.

L'invention a également pour objet un polypeptide comprenant tout ou partie de la séquence peptidique SEQ ID n°2 ou d'un dérivé de celle-ci modifiée par mutation, substitution, délétion et/ou modification d'un ou plusieurs résidus, et possédant la capacité de lier la galanine ou un variant de la galanine, ledit dérivé étant tel que la galanine 2-29 et la galanine 1-16 inhibent compétitivement la liaison de la (¹²⁵I) galanine avec une affinité plus faible que la galanine 1-29 et que la galanine 2-29 inhibe compétitivement !a liaison de la (¹²⁵I) galanine avec une affinité plus faible que la galanine 1-16.

De tels dérivés peuvent être générés dans des buts différents, tels que notamment celui d'augmenter l'affinité du peptide pour son(ses) ligand(s), celui d'améliorer ses niveaux de production, celui d'augmenter sa résistance à des protéases, celui d'augmenter et/ou de modifier son activité, ou celui de lui conférer de nouvelles propriétés pharmacocinétiques et/ou biologiques. Parmi les dérivés résultant d'une addition, on peut citer par exemple les polypeptides chimères comportant une partie hétérologue supplémentaire liée à une extrémité. Le terme dérivé comprend également les polypeptides homologues au polypeptide SEQ ID n° 1, issus d'autres sources cellulaires et notamment de cellules d'origine humaine, ou d'autres organismes, et possédant une activité de même type. De tels polypeptides homologues comprennent notamment les différents sous-types de récepteurs galaninergiques. Ils peuvent être obtenus en particulier par des expériences d'hybridation comme décrit dans les exemples.

Préférentiellement, les polypeptides de l'invention sont des polypeptides possédant la capacité de lier la galanine ou un variant de la galanine. Toujours selon un mode préféré, les polypeptides de l'invention sont susceptibles d'être reconnus par des anticorps reconnaissant la séquence peptidique SEQ ID n° 1 complète. De tels anticorps peuvent être générés par toute technique connue de l'homme du métier, en utilisant comme antigènes les polypeptides décrits dans la présente demande.

Comme indiqué dans les exemples, ces polypeptides peuvent être exprimés dans différents types cellulaires pour former des récepteurs galaninergiques fonctionnels.

Les polypeptides de l'invention peuvent être obtenus par expression dans un hôte cellulaire d'une séquence nucléotidique telle que décrite ci-dessus par synthèse chimique, sur la base de la séquence SEQ ID n° 1 en utilisant les techniques connues de l'homme du métier, ou par une combinaison de ces techniques.

Un autre objet de l'invention concerne les cellules recombinées capables d'exprimer à leur surface un polypeptide ayant une activité de récepteur galaninergique. Ces cellules peuvent être obtenues par introduction d'une séquence nucléotidique telle que définie ci-dessus, puis culture desdites cellules dans des conditions d'expression de ladite séquence.
Les cellules recombinées selon l'invention peuvent être aussi bien des cellules eucaryotes que procaryotes. Parmi les cellules eucaryotes qui conviennent, on peut citer les cellules animales, les levures, ou les champignons. En particulier, s'agissant de levures, on peut citer les levures du genre *Saccharomyces, Kluyveromyces*, *Pichia*, *Schwanniomyces*, ou *Hansenula.* S'agissant de cellules animales, on peut citer les cellules COS, CHO, C127, NIH-3T3, etc. Parmi les champignons, on peut citer plus particulièrement *Aspergillus* ssp. ou *Trichoderma* ssp. Comme cellules procaryotes, on préfère utiliser les bactéries suivantes *E.col*i, *Bacillus*, ou *Streptomyces*. Les cellules ainsi obtenues peuvent être utilisées pour mesurer la capacité de différentes molécules à se comporter comme ligand ou comme modulateur de l'activité des récepteurs galaninergiques. Plus particulièrement, elles peuvent ainsi être utilisées dans un procédé de mise en évidence et d'isolement de ligands ou de modulateur de l'activité des récepteurs galaninergiques, et, plus préférentiellement, d'agonistes et d'antagonistes.
Un autre objet de l'invention concerne donc un procédé de mise en évidence et/ou d'isolement de ligands des récepteurs galaninergiques, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide ayant une activité de récepteur galaninergique dans des conditions permettant l'interaction entre ledit polypeptide et ladite molécule dans le cas où celle-ci posséderait une affinité pour ledit polypeptide, et,
- on détecte et/ou isole les molécules liées au dit polypeptide.
Dans un mode particulier, ce procédé de l'invention est adapté à la mise en évidence et/ou l'isolement d'agonistes et d'antagonistes de la galanine pour les récepteurs galaninergiques.

Un autre objet de l'invention concerne un procédé de mise en évidence et/ou d'isolement de modulateurs des récepteurs galaninergiques, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide ayant une activité de récepteur galaninergique, en présence du ligand endogène dudit récepteur, dans des conditions permettant l'interaction entre ledit polypeptide et son ligand, et,
- on détecte et/ou isole les molécules capables de moduler l'activité du ligand sur ledit polypeptide.
   Dans un mode particulier, ce procédé de l'invention est adapté à la mise en évidence et/ou l'isolement de modulateurs de l'activité de la galanine sur les récepteurs galaninergiques.

Un autre objet de l'invention concerne l'utilisation d'un ligand ou d'un modulateur identifié et/ou obtenu selon le procédé décrit ci-avant comme médicament. De tels ligands ou modulateurs peuvent en effet permettre de traiter certaines affections liées aux récepteurs galaninergiques. En particulier, les récepteurs galaninergiques étant les médiateurs d'un effet analgésique, les agonistes de ces récepteurs peuvent être utilisés pour diminuer les sensations de douleur. D'autres activités des récepteurs galaninergiques ont été mentionnées en introduction.

L'invention concerne également tout médicament comprenant comme principe actif au moins une molécule agissant sur un récepteur de l'invention. Préférentiellement la molécule est un ligand ou un modulateur identifié et/ou isolé selon le procédé décrit précédemment.

D'autres avantages de la présente invention apparaîtront à la lecture des exemples qui suivent, qui doivent être considérés comme illustratifs et non limitatifs.

### Légende des figures

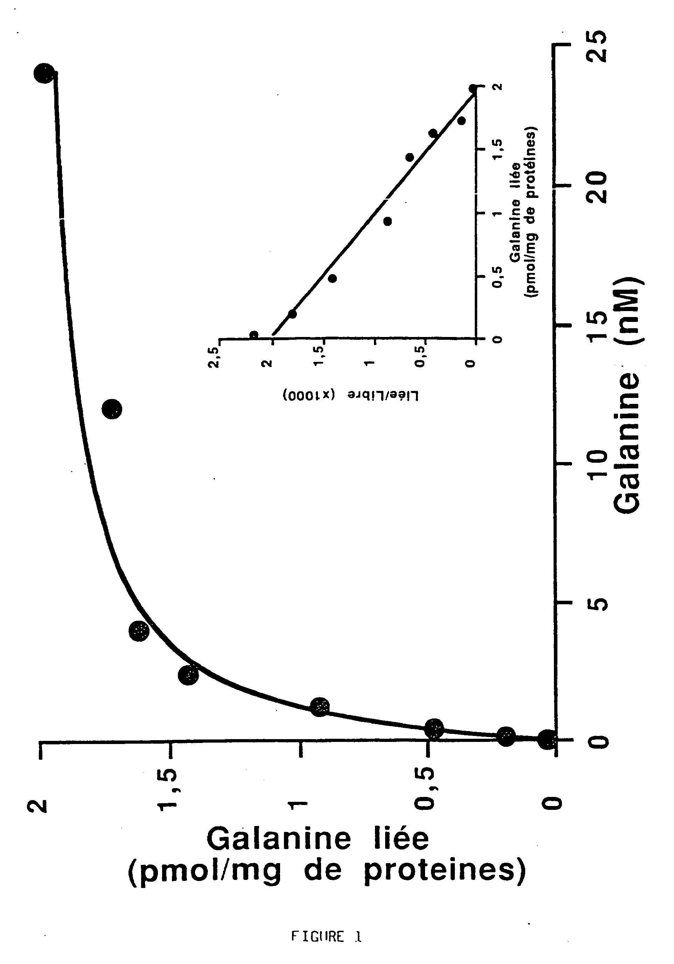

Figure 1: Saturation des récepteurs à la galanine par la galanine de porc, sur les membranes de cellules Cos1 transfectées par le clone GalR1. L' encart représente la transformation de Scatchard des données de la courbe de liaison (Les valeurs présentées correspondent à une expérience représentative où chaque point a été déterminé en triplicat).

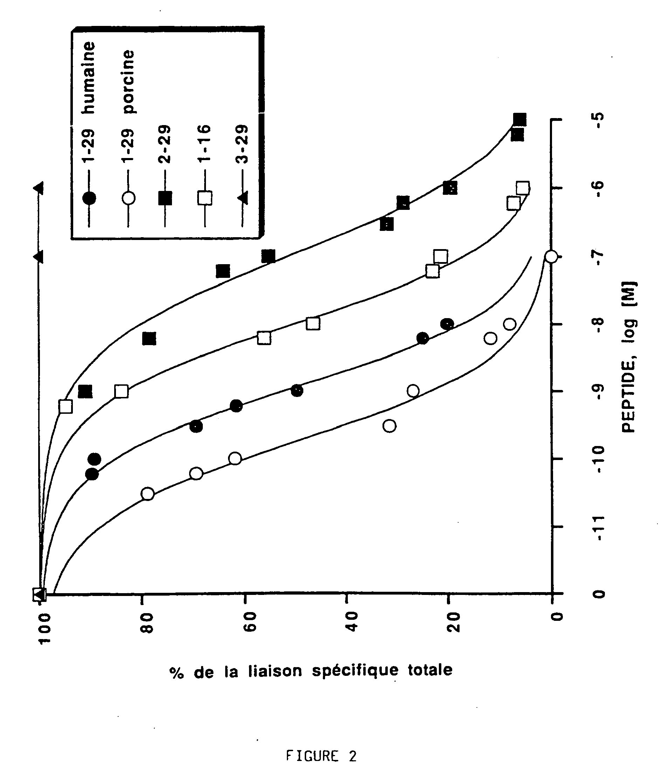

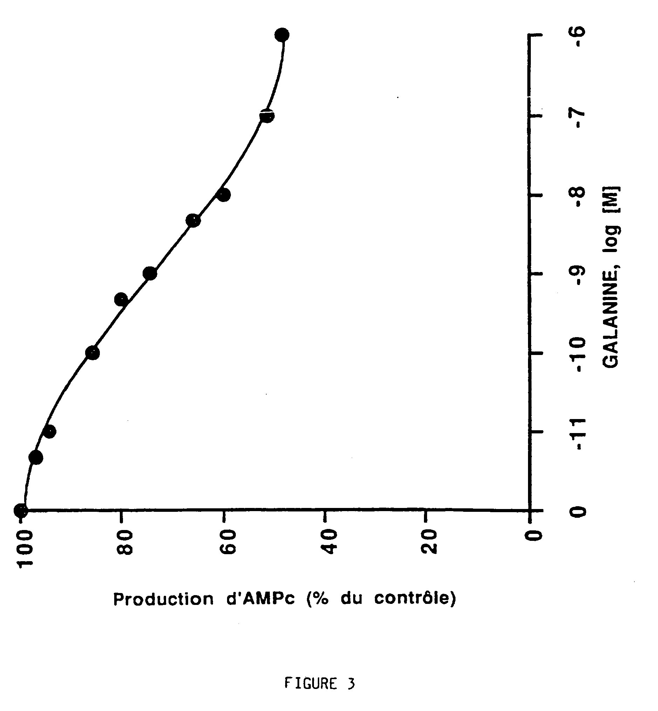

Figure 2: Déplacements par des peptides de la liaison spécifique de la (¹²⁵I)-galanine aux membranes de cellules Cos1 transfectées par le clone GalR1 (Les valeurs présentées correspondent à une expérience représentative où chaque point a été déterminé en triplicat).
Figue 3: Inhibition par la galanine de la stimulation de la production d'AMPc dans des cellules Cos1 transfectées par le clone GALR1 (Les valeurs présentées correspondent à une expérience représentative où chaque point a été déterminé en duplicat).

### Techniques générales de colonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans *Escherichia coli,* etc, sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].
Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 selon les recommandations du fournisseur.
Le remplissage des extrémités 5' proéminentes est effectué par le fragment de Klenow de l'ADN Polymérase I d*'E. coli* selon les spécifications du fournisseur. La destruction des extrémités 3' proéminentes est effectuée en présence de l'ADN Polymérase du phage T4 utilisée selon les recommandations du fabricant. La destruction des extrémités 5' proéminentes est effectuée par un traitement ménagé par la nucléase S1.
La mutagénèse dirigée *in vitro* par oligodéoxynucléotides synthétiques est effectuée selon la méthode développée par Taylor et al. [Nucleic Acids Res. 13 (1985) 8749-8764].
L'amplification enzymatique de fragments d'ADN par la technique dite de PCR [Polymérase-catalyzed Chain Reaction, Saiki R.K. et al., Science 230 (1985) 1350-1354 ; Mullis K.B. et Faloona F.A., Meth. Enzym. 155 (1987) 335-350].
La vérification des séquences nucléotidiques est effectuée par la méthode développée par Sanger et al. [Proc. Natl. Acad. Sci. USA, 74 (1977) 5463-5467].

Pour les expériences d'hybridation, les conditions de stringence sont basées sur Maniatis T. et al., précitée.

### 1. Isolement du récepteur humain à la galanine

Cet exemple décrit l'isolement du clone GalRI codant pour le récepteur à la galanine humain par criblage par expression d'une banque d'ADNc.

### 1a. Construction de la banque

Une banque d'ADNc a été réalisée à partir de la lignée mélanocytaire humaine Bowes (ATCC CRL 9607), en utilisant le vecteur d'expression transitoire de mammifère pcDNA1. Après extraction des ARN totaux, les ARN polyA⁺ sont purifiés sur colonne d' oligodT cellulose. La synthèse d'ADNc est effectuée en présence d'un mélange d'amorces (oligodT et oligonucléotides s'hybridant au hasard). Après un fractionnement par la taille (>1100 paires de bases), la banque a été construite par ligature des cDNA dans le vecteur pcDNA1 (sites de clonage Bstx1) et transformation dans *E. Coli* ( MC 1061/P3). 10⁶ clones indépendants ont été obtenus et amplifiés. Environ 720000 clones bactériens ont alors été cultivés en milieu solide sélectif à une densité de 8000 colonies par boite. 90 lots d'ADN plasmidique, correspondant chacun à 8000 colonies, ont été préparés par lyse alcaline.

### 1b. Transfection

L'ADN plasmidique de chaque lot a été transfecté indépendemment dans les cellules Cos1 (ATCC CRL 1650) par électroporation selon le protocole suivant: 32 mg d'ADN, 8 10⁶ cellules dans 500 ml de milieu DMEM (Dulbecco Modified Eagle Medium) supplémenté par 10% de sérum de veau foetal, 6 mM de glutamine, 100UI/ml de pénicilline et 100 mg/ml de streptomycine sous 5 % de CO2, 960 mFarad, 250 Volts, électroporateur Biorad. Après électroporation, les cellules ont été cultivées en monocouche dans des boites de culture (Nunc) de 16 cm de diamètre pendant 72 heures. Dans ces conditions, une moyenne de 30 % de cellules sont transfectées.

### 1c. Criblage par expression

Les cellules transfectées ont été testées pour leur capacité à lier la (¹²⁵I)-galanine. 48 heures après la transfection, les cellules ont été changées. Le jour de la liaison, elles sont mises en présence de DMEM pendant 1 heure, puis lavées par 10 ml de tampon 1 (Tris HCl 25 mM pH: 7,5, MgCl2 10mM, BSA 2 %). Les cellules ont alors été incubées pendant 5 heures à 20°C en présence de tampon 2 (tampon 1 contenant un cocktail d'inhibiteurs de protéases:10 mg/ml d' aprotinine, 1mg/ ml de bacitracine, 10 mg/ml de pepstatine A et 10⁻⁵M de phénylméthylsulfonylfluoride) contenant 10⁻¹⁰M de (¹²⁵I)-galanine (NEN, galanine 1-29 de porc, AS: 2200 curie/mmole). Après lavage (5x15 ml de tampon 1), les boîtes ont été séchées et exposées en autoradiographie (film Amersham hyperscreen MP) pendant 15 jours à -80°C.

### 1d. Isolement du clone GalR1 codant pour le récepteur à la galanine

Sur les 720000 clones bactériens testés, un lot de 8000 colonies, donnant un signal spécifique en autoradiographie (i.e (i) dépassant le bruit de fond, (ii) disparaissant en présence de 10⁻⁶ M de galanine froide) a été identifié. Ce lot a ensuite été divisé en 48 lots de 700 clones chacun. Après transfection, les cellules ont été testées pour leur capacité à lier la (¹²⁵I)-galanine. Trois lots produisent un signal spécifique en autoradiographie. L'un d'eux a été sous-divisé 2 fois (25 lots de 30 clones puis 60 clones individuels), conduisant à 2 clones conférant aux cellules Cos1 une forte capacité à lier de la (¹²⁵I)-galanine.

### 2. Etude structurale du récepteur à la galanine

Les 2 clones isolés dans l'exemple 1 contiennent un insert identique de 3,1 kb. La séquence d'un des 2 clones a été déterminée sur les 2 brins, par la technique fluorescente (Applied Biosystems) dérivée de la méthode de Sanger, en utilisant la Taq Polymérase et des didéoxynucléotides fluorescents.

La séquence obtenue correspond à la séquence SEQ ID n°1. Elle porte une phase ouverte de lecture de 1053 paires de bases: le site d'initiation de la traduction est attribué au codon ATG en position 787 de la séquence SEQ ID n°1.
La phase ouverte de lecture ainsi définie code pour un protéine de 349 acides aminés (SEQ ID n°1) de poids moléculaire calculé de 38897 Daltons. L'analyse du profil d'hydrophaticité de la séquence en acides aminés suggère que le récepteur possède 7 segments constitués d'acides aminés majoritairement hydrophobes, formant globalement des segments transmembranaires (STM), point commun aux membres de la famille des récepteurs couplés aux protéines G. Le récepteur est organisé comme suit: MET1-ASN33, segment N-terminal; PHE34-LEU58, STM I ; ALA59-ASN71, boucle intracellulaire I ; LEU72-LEU98, STM II ; PRO99-LYS109, boucle extracellulaire I ; PHE110-ASP132, STM III ; ARG133-ASN151, boucle intracellulaire II ; ALA152-VAL170, STM IV ; ALA171-ALA199, boucle extracellulaire II ; TYR200-VAL223, STMV ; LEU224-LYS244, boucle intracellulaire III ; THR245-LEU268, STM VI ; TRP269-PRO279, boucle extracellulaire III ; ALA280-SER307, STMVII ; GLU308-VAL349, segment C-terminal.
De plus, certains acides aminés conservés au sein des membres de la famille des récepteurs couplés aux protéines G sont présents dans la séquence protéique du récepteur GALR1. En particulier, on note (1) 2 sites de N-glycosylation (ASN7 et ASN12), (2) 1 site de phosphorylation par la protéine kinase cAMP ou cGMP dépendante (SER143), (3) 1 site de famésylation (CYS340), (4) 2 cystéines (CYS187 et 308), (5) 4 prolines (PRO169,212,262 et300), (6) le motif GLY.ASN.X.X.VAL (50-54), (7) le motif ASP.ARG.TYR (132-134), (8) le motif ASN.PRO.ILE.X.TYR (299-303). La séquence de la protéine de la présente invention a finalement été comparée avec les séquences des protéines de la famille des récepteurs couplés aux protéines G (banque de données Swissprot data). L'homologie la plus importante (33,8%, incluant 3 brèches) est observée avec le récepteur à la somatostatine de type 4.

### 3._ Etudes biochimiques du récepteur GalR1 exprimé transitoirement dans les cellules Cos1

### 3a. Stoechiométrie et pharmacologie du récepteur GalR1

L'ADN plasmidique du clone GalR1 a été péparé par les techniques de biologie moléculaire classiques pour transfecter des cellules Cos1. Les membranes des cellules transfectées ont ensuite été utilisées pour estimer les paramètres de liaison à l'équilibre de la galanine 1-29 et de fragments de galanine.
L'ADN plasmidique (90 mg/8 10⁶ cellules Cos1) a été transfecté par électroporation comme décrit précédemment (1b). 72 heures après la transfection, les cellules recombinantes ont été récoltées et sédimentées. Les membranes ont alors été préparées comme suit : à 4°C, le culot cellulaire est mis en présence de 10 ml de tampon 3 (Hepes 5 mM, pH 7,5) pendant 20 minutes ; après centrifugation à 13 000 g pendant 15 minutes, le culot est resuspendu dans le tampon 3 et conservé à -80°C jusqu'à utilisation.
Des expériences de liaison à l'équilibre (saturation et compétition) ont été réalisées sur les membranes en présence de (¹²⁵I)-galanine et de différents peptides dans les conditions expérimentales suivantes : les membranes (10 mg de proteine) ont été incubées pendant 30 minutes à 20°C avec 5 10⁻¹¹M de (¹²⁵I)-galanine en absence ou en présence de concentrations croissantes de galanine 1-29, galanine 2-29, galanine 3-29 ou galanine 1-16 (10⁻¹¹M-10⁻⁶M) dans un volume final de 0,25 ml de tampon 2 (1c). La réaction a ensuite été stoppée par addition de 0,25 ml de tampon 1 (1c) et centrifugation pendant 15 minutes à 13000 g. Le culot obtenu a été lavé trois fois avec 0,2 ml de tampon Tris 25 mM (pH: 7,5) glacé contenant 10 % de sucrose, puis la radioactivité a été mesurée avec un compteur à scintillation gamma. La liaison non spécifique est mesurée en présence de 10⁻⁷ M de galanine 1-29 de porc. Les valeurs des constantes d'affinité (Ki) ont été obtenues en suivant l'équation de Cheng et Prussof: Ki = IC50/ (1 + L/Kd).

Dans les conditions expérimentales ci-dessus décrites, les résultats suivants sont observés : contrairement aux cellules Cos1 non transfectées, ou transfectées par un plasmide contrôle, les cellules Cos1 transfectées par le clone GalR1 présentent une liaison spécifique de la galanine iodée. L'analyse des résultats par la méthode de Scatchard montre l'existence d'une seule classe de sites de haute affinité, avec une constante de dissociation apparente (Kd) = 0,8 nM et de haute capacité, avec un nombre maximal de sites de fixation (Bmax) = 2,8 pmoles/mg proteines. Compte tenu de l'efficacité de transfection (environ 30 %, 1b), le niveau d'expression de récepteurs galaninergiques de l'invention dans les cellules Cos1 est estimé à 5 10⁵ sites/cellule.
Les galanines de rat, de porc et humaine inhibent de façon compétitive la liaison de la (¹²⁵I)-Galanine aux membranes des cellules transfectées avec un Ki = 0,3, 0,2 et 0,8 nM respectivement. La galanine 2-29 (Ki= 115 nM) et la galanine 1-16 (Ki= 5 nM) inhibent compétitivement la liaison de la (¹²⁵I)-galanine avec une affinité plus faible que la galanine 1-29. Ce clone n'a pas d'affinité pour la galanine 3-29.

L'ordre d'efficacité de ces différentes molécules confirme la spécificité du récepteur GalR1 comme récepteur galaninergique. Ces résultats montrent par ailleurs que les cellules Cos1 de la présente invention sont capables d'exprimer le récepteur à la galanine ayant des caractéristiques de liaison comparables à celles du récepteur natif.

### 3b. Etude fonctionnelle du récepteur GalR1

Le couplage du récepteur Galanine à l'adénylate cyclase a été estimé par la capacité de la galanine à inhiber la production d'AMPc dans les cellules Cos1 transfectées par le clone GalR1. Pour cette étude, 10⁵ cellules transfectées dans les conditions décrites ci-dessus ont été ensemencées dans des plaques de culture multipuits (1,5 cm de diamètre) et les expériences d'AMPc ont été réalisées 72 heures après la transfection (le milieu de culture est renouvelé la veille de l'expérience). Après une préincubation de 1 heure à 37°C en milieu DMEM, les cellules ont été lavées par du DMEM contenant 2 % de BSA. Elles ont alors été incubées 30 minutes à 37°C dans ce même milieu contenant 2 10⁻⁴M d'isobutylmethylxanthine, en absence et en présence de forskoline (10⁻⁴M) et/ou de galanine humaine (10⁻¹¹M-10⁻⁶M). Après trois lavages des cellules en milieu DMEM, l'AMPc intracellulaire est extrait par additon d'ethanol 70 %. Après lyophylisation, l'AMPc est mesuré par radioimmunologie (Kit AMPc, Amersham).
Les résultats montrent que dans les cellules Cos1 transfectées par le clone GalR1, la galanine inhibe fortement la production d'AMPc intracellulaire induite par la forskoline (la production induite est de l'ordre de 2 pmoles/10⁵ cellules, soit 10 fois le basal). Cet effet n'est pas observé dans des conditions basales. La demi-inhibition et l'inhibition maximale de la production d'AMPc sont induites par 10⁻⁹M et 10⁻⁶M de galanine humaine, respectivement. En présence de 10⁻⁶M de galanine, la production d'AMPc intracellulaire induite par la forskoline est diminuée de 50 %.

L'inhibition par la galanine de la production d'AMPc intracellulaire induite par la forskoline confirme la spécificité du récepteur GalR1 comme récepteur galaninergique. Ces résultats montrent par ailleurs que les cellules Cos1 de la présente invention sont capables d'exprimer le récepteur à la galanine ayant des caractéristiques fonctionnelles comparables à celles du récepteur natif.

### 4. Recherches de séquences homologues, notamment de sous-tyes dans d'autres tissus

Cet exemple montre que la séquence nucléotidique SEQ ID n°1 ou des fragments de celle-ci peuvent être utilisés pour la mise en évidence de séquences homologues, notamment de sous-types, à partir d'autres tissus. Pour cela, différentes techniques sont utilisables, telles que la PCR, l' hybridation *in situ,* le transfert de Northern, etc...
Plus particulièrement, pour la recherche par PCR, les ARN totaux des différents tissus étudiés sont préparés, puis soumis à réverse transcription et à une amplification, en présence de transcriptase réverse, de Taq polymérase et des amorces dérivées de la séquence SEQ ID n°1 appropriées. Les produits ainsi obtenus sont ensuite transférés sur filtres de nitrocellulose et hybridés dans des conditions de stringence variables.
Les séquences homologues mises en évidence lors de ces expériences peuvent être ensuite isolées et/ou amplifiées par les techniques classiques de biologie moléculaire.

## Revendications

1. Séquence nucléotidique codant pour un polypeptide ayant une activité de récepteur galaninergique, **caractérisée en ce qu'**elle est choisie parmi :
(a) tout ou partie de la séquence nucléotidique SEQ ID n° 1, et
(b) les séquences dérivées des séquences (a) en raison de la dégénérescence du code génétique.

2. Séquence nucléotidique selon la revendication 1 **caractérisée en ce qu'**elle code pour un récepteur galaninergique humain.

3. Séquence nucléotidique **caractérisée en ce qu'**elle comprend la séquence nucléotidique SEQ ID n° 1 ou son brin complémentaire.

4. Séquence nucléotidique selon les revendications 1 à 3 **caractérisée en ce qu'**elle est choisie parmi les séquences d'ADN génomique, d'ADNc, d'ARN, les séquences hybrides ou les séquences synthétiques ou semi-synthétiques.

5. Séquence selon l'une des revendications 1 à 4 **caractérisée en ce que** la partie codant pour ledit polypeptide est placée sous le contrôle de signaux permettant son expression dans un hôte cellulaire.

6. Polypeptide ayant une activité de récepteur galaninergique résultant de l'expression d'une séquence nucléotidique, ladite séquence étant choisise parmi :
a) tout ou partie de la séquence nucléotidique SEQ ID n° 1, et
b) les séquences dérivées des séquences (a) en raison de la dégénérescence du code génétique.

7. Polypeptide comprenant tout ou partie de la séquence peptidique SEQ ID n° 2 ou d'un dérivé de celle-ci, modifiée par mutation, substitution, délétion et/ou modification d'un ou plusieurs résidus, et possédant la capacité de lier la galanine ou un variant de la galanine, ledit dérivé étant tel que la galanine 2-29 et la galanine 1-16 inhibent compétitivement la liaison de la (¹²⁵I) galanine avec une affinité plus faible que la galanine 1-29 et que la galanine 2-29 inhibe compétitivement la liaison de la (¹²⁵I) galanine avec une affinité plus faible que la galanine 1-16.

8. Séquence antisens capable d'inhiber au moins partiellement la production d'un polypeptide selon l'une des revendications 6 ou 7.

9. Cellule recombinée exprimant à sa surface un polypeptide selon l'une des revendications 6 ou 7, obtenue par introduction d'une séquence selon la revendication 5.

10. Cellule selon la revendication 9, **caractérisée en ce qu'**elle est choisie parmi les cellules eucaryotes ou procaryotes.

11. Procédé de mise en évidence et/ou d'isolement de ligands des récepteurs galaninergiques, **caractérisé en ce que** l'on réalise les étapes suivantes :
- on met en contact une molécule ou un mélange contenant différentes molécules, éventuellement non-identifiées, avec une cellule recombinée selon la revendication 9 exprimant à sa surface un polypeptide selon l'une des revendications 6 et 7, dans des conditions permettant l'interaction entre ledit polypeptide et ladite molécule dans le cas où celle-ci posséderait une affinité pour ledit polypeptide, et,
- on détecte et/ou isole les molécules liées au dit polypeptide.

12. Procédé selon la revendication 11 pour la mise en évidence et/ou l'isolement d'agonistes et/ou d'antagonistes des récepteurs galaninergiques.

13. Procédé de mise en évidence et/ou d'isolement de modulateurs des récepteurs galaninergiques, **caractérisé en ce que** l'on réalise les étapes suivantes :
- on met en contact une molécule ou un mélange contenant différentes molécules, éventuellement non-identifiées, avec une cellule recombinée selon la revendication 9 exprimant à sa surface un polypeptide, selon l'une des revendications 6 et 7, ayant une activité de récepteur galaninergique, en présence d'un ligand dudit récepteur, dans des conditions permettant l'interaction entre ledit polypeptide et le ligand, et,
- on détecte et/ou isole les molécules capables de moduler l'activité du ligand sur ledit polypeptide.

14. Anticorps reconnaissant un polypeptide selon la revendication 6.

15. Procédé d'obtention d'un polypeptide selon l'une des revendications 6 et 7 comprenant l'expression d'une séquence nucléotidique selon l'une des revendications 1 à 5 dans une cellule.

## Claims

1. Nucleotide sequence encoding a polypeptide having galaninergic receptor activity, **characterized in that** it is chosen from:
(a) all or part of the nucleotide sequence SEQ ID No. 1, and
(b) the sequences derived from the sequences (a) due to the degeneracy of the genetic code.

2. Nucleotide sequence according to Claim 1, **characterized in that** it encodes a human galaninergic receptor.

3. Nucleotide sequence, **characterized in that** it comprises the nucleotide sequence SEQ ID No. 1 or the strand complementary thereto.

4. Nucleotide sequence according to Claims 1 to 3, **characterized in that** it is chosen from genomic DNA, cDNA or RNA sequences, hybrid sequences or synthetic or semi-synthetic sequences.

5. Sequence according to one of Claims 1 to 4,
**characterized in that** the part encoding said polypeptide is placed under the control of signals which allow its expression in a cellular host.

6. Polypeptide having galaninergic receptor activity, resulting from the expression of a nucleotide sequence, said sequence being chosen from:
a) all or part of the nucleotide sequence SEQ ID No. 1, and
b) the sequences derived from the sequences (a) due to the degeneracy of the genetic code.

7. Polypeptide comprising all or part of the peptide sequence SEQ ID No. 2 or of a derivative thereof, modified by mutation, substitution, deletion and/or modification of one or more residues, and having the ability to bind galanin or a galanin variant, said derivative being such that galanin 2-29 and galanin 1-16 competitively inhibit the binding of (¹²⁵I) galanin with a lower affinity than galanin 1-29, and that galanin 2-29 competitively inhibits the binding of (¹²⁵I) galanin with a lower affinity than galanin 1-16.

8. Antisense sequence capable of at least partially inhibiting the production of a polypeptide according to either of Claims 6 and 7.

9. Recombined cell expressing, at its surface, a polypeptide according to either of Claims 6 or 7, obtained by introducing a sequence according to Claim 5.

10. Cell according to Claim 9, **characterized in that** it is chosen from eukaryotic or prokaryotic cells.

11. Method for demonstrating and/or isolating galaninergic receptor ligands, **characterized in that** the following steps are carried out:
- a molecule or a mixture containing various molecules, possibly unidentified, is brought into contact with a recombined cell according to Claim 9 expressing, at its surface, a polypeptide according to either of Claims 6 and 7, under conditions which allow interaction between said polypeptide and said molecule in the case where the latter has an affinity for said polypeptide, and
- detecting and/or isolating the molecules bound to said polypeptide.

12. Method according to Claim 11, for demonstrating and/or isolating galaninergic receptor agonists and/or antagonists.

13. Method for demonstrating and/or isolating galaninergic receptor modulators, **characterized in that** the following steps are carried out:
- a molecule or a mixture containing various molecules, possibly unidentified, is brought into contact with a recombined cell according to Claim 9 expressing, at its surface, a polypeptide according to either of Claims 6 and 7, having galaninergic receptor activity, in the presence of a ligand of said receptor, under conditions which allow interaction between said polypeptide and the ligand, and
- detecting and/or isolating the molecules capable of modulating the activity of the ligand on said polypeptide.

14. Antibody which recognizes a polypeptide according to Claim 6.

15. Method for obtaining a polypeptide according to either of Claims 6 and 7, comprising the expression of a nucleotide sequence according to one of Claims 1 to 5 in a cell.

## Patentansprüche

1. Nukleotidsequenz, die für ein Polypeptid mit einer Aktivität eines galaninergen Rezeptors codiert, **dadurch gekennzeichnet, dass** sie ausgewählt ist aus:
(a) der gesamten oder einem Teil der Nukleotidsequenz SEQ ID NR: 1 und
(b) den Sequenzen, die von den Sequenzen (a) aufgrund der Degeneration des genetischen Codes abgeleitet sind.

2. Nukleotidsequenz nach Anspruch 1, **dadurch gekennzeichnet, dass** sie für einen humanen galaninergen Rezeptor codiert.

3. Nukleotidsequenz, **dadurch gekennzeichnet, dass** sie die Nukleotidsequenz SEQ ID NR: 1 oder deren komplementären Strang umfasst.

4. Nukleotidsequenz nach den Ansprüchen 1 bis 3, **dadurch gekennzeichnet, dass** sie aus genomischen DNA-, cDNA-, RNA-, Hybrid-Sequenzen oder synthetischen oder semisynthetischen Sequenzen ausgewählt ist.

5. Sequenz nach einem der Ansprüche 1 bis 4, **dadurch gekennzeichnet, dass** der Teil, der für das Polypeptid codiert, unter die Kontrolle von Signalen gestellt wird, die seine Expression in einem zellulären Wirt gestatten.

6. Polypeptid mit der Aktivität eines galaninergen Rezeptors, das aus der Expression einer Nukleotidsequenz hervorgeht, wobei die Sequenz ausgewählt ist aus:
(a) der gesamten oder einem Teil der Nukleotidsequenz SEQ ID NR: 1 und
(b) den Sequenzen, die von den Sequenzen (a) aufgrund der Degeneration des genetischen Codes abgeleitet sind.

7. Polypeptid, umfassend die gesamte oder einen Teil der Peptidsequenz SEQ ID NR: 2 oder ein(es) Derivat(es) davon, das durch Mutation, Substitution, Deletion und/oder Modifikation eines oder mehrere Reste modifiziert ist, und mit der Fähigkeit, Galanin oder eine Galanin-Variante zu binden, wobei das Derivat derart ist, dass Galanin 2-29 und Galanin 1-16 die Bindung von (¹²⁵I)-Galanin mit einer schwächeren Affinität als Galanin 1-29 kompetitiv hemmen und dass Galanin 2-29 die Bindung von (¹²⁵I)-Galanin mit einer schwächeren Affinität als Galanin 1-16 kompetitiv hemmt.

8. Antisense-Sequenzen, welche die Produktion eines Polypeptids nach einem der Ansprüche 6 oder 7 wenigstens teilweise hemmen können.

9. Rekombinante Zelle, die auf ihrer Oberfläche ein Polypeptid nach einem der Ansprüche 6 oder 7 exprimiert und durch Einbringen einer Sequenz nach Anspruch 5 erhalten wird.

10. Zelle nach Anspruch 9, **dadurch gekennzeichnet, dass** sie aus eukaryontischen oder prokaryontischen Zellen ausgewählt ist.

11. Verfahren zum Nachweis und/oder zur Isolation von Liganden galaninerger Rezeptoren, **dadurch gekennzeichnet, dass** man in den folgenden Schritten:
- ein Molekül oder ein Gemisch, das verschiedene, gegebenenfalls nicht identifizierte, Moleküle enthält, mit einer rekombinanten Zelle nach Anspruch 9, die auf ihrer Oberfläche ein Polypeptid nach einem der Ansprüche 6 und 7 exprimiert, unter Bedingungen in Kontakt bringt, welche die Wechselwirkung zwischen dem Polypeptid und dem Molekül gestatten, wenn dieses eine Affinität für das Polypeptid besitzt, und
- die an das Polypeptid gebundenen Moleküle nachweist und/oder isoliert.

12. Verfahren nach Anspruch 11 zum Nachweis und/oder zur Isolation von Agonisten und/oder Antagonisten galaninerger Rezeptoren.

13. Verfahren zum Nachweis und/oder zur Isolation von Modulatoren galaninerger Rezeptoren, **dadurch gekennzeichnet, dass** man in den folgenden Schritten:
- ein Molekül oder ein Gemisch, das verschiedene, gegebenenfalls nicht identifizierte, Moleküle enthält, mit einer rekombinanten Zelle nach Anspruch 9, die auf ihrer Oberfläche ein Polypeptid nach einem der Ansprüche 6 und 7 mit einer Aktivität eines galaninergen Rezeptors exprimiert, in Gegenwart eines Liganden des Rezeptors unter Bedingungen in Kontakt bringt, welche die Wechselwirkung zwischen dem Polypeptid und dem Liganden gestatten, und
- die Moleküle nachweist und/oder isoliert, die zur Modulation der Aktivität des Liganden an dem Polypeptid fähig sind.

14. Antikörper, der ein Polypeptid nach Anspruch 6 erkennt.

15. Verfahren zum Erhalten eines Polypeptids nach einem der Ansprüche 6 und 7, umfassend die Expression einer Nukleotidsequenz nach einem der Ansprüche 1 bis 5 in einer Zelle.
